# FASCICULE DE BREVET EUROPEEN

(11) **EP 0 557 192 B1**
(45) Date de publication et mention de la délivrance du brevet: **21.08.1996**
(21) Numéro de dépôt: 93400404.5
(22) Date de dépôt: 18.02.1993
(51) Int. Cl.: C07C 69/747, C07C 33/46, A01N 53/00, A01N 37/44, A01N 37/10, C07C 69/612, C07C 229/42, C07C 255/39

(54) **Esters pyréthrinoides, dérivés de l'alcool 6-(trifluorométhyl)benzylique, leur procédé de préparation et leur application comme pesticides**
Von 6-Trifluoromethylbenzylalkohol abgeleitete Pyrethrinoidester, Verfahren zu ihrer Herstellung und ihre Anwendung als Pestizide
Pyrethrinoid esters derived from 6-trifluoromethyl benzyl alcohol, process for their preparation and their use as pesticides

(30) Priorité: 21.02.1992 FR 9202010
(43) Date de publication de la demande: 25.08.1993
(73) Titulaire: ROUSSEL UCLAF, 93230 Romainville (FR)
(72) Inventeur: Babin, Didier, F-78180 Montigny (FR); Benoit, Marc, F-13360 Roquevaire (FR); Demoute, Jean-Pierre, F-93360 Neuilly Plaisance (FR)
(74) Mandataire: Vieillefosse, Jean-Claude

(56) Documents cités:
- EP-A- 0 007 421
- EP-A- 0 008 732
- EP-A- 0 032 121
- EP-A- 0 050 534
- EP-A- 0 181 284
- FR-A- 2 147 310
- GB-A- 2 058 065

## Description

La présente invention concerne de nouveaux esters pyréthrinoïques, dérivés de l'alcool 6-(trifluorométhyl) benzylique, leur procédé de préparation et leur application comme pesticides.

On connaissait des esters benzyle de l'acide 2,2-diméthyl cyclopropane carboxylique présentant des produits pesticides cf. FR-A-2147310 et EP-A-50534. L'invention a pour objet sous toutes les formes stéréoisomères possibles ainsi que leurs mélanges, les composés de formule (I) : 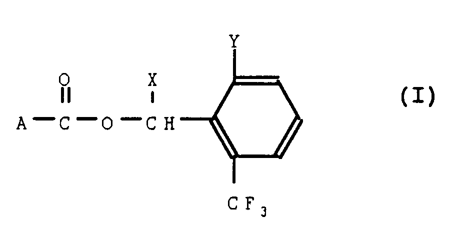 dans laquelle :
- X représente un atome d'hydrogène, un radical alkyle, alkényle ou alkynyle renfermant jusqu'à 4 atomes de carbone ou un radical cyano ou un radical aralkynyle renfermant jusqu'à 10 atomes de carbone,
   Y représente un atome d'halogène ou un radical CH₂F, CHF₂ ou CF₃,

### ou bien A représente un radical :

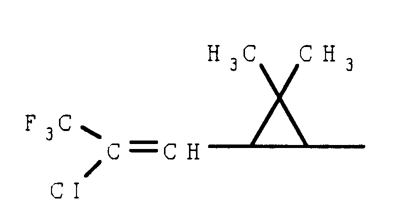

### ou bien A représente un radical :

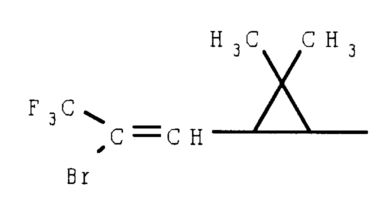

### ou bien A représente un radical :

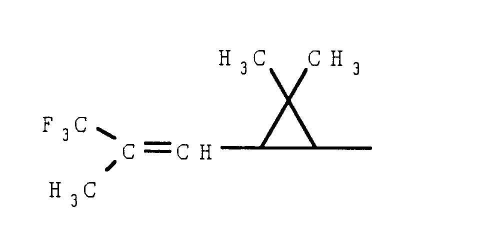

- soit Z₂ représente un radical : 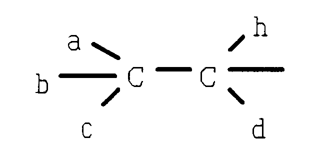 dans lequel a, b, c et d, identiques ou différents représentent chacun un atome d'halogène,
- soit Z₂ représente un radical : 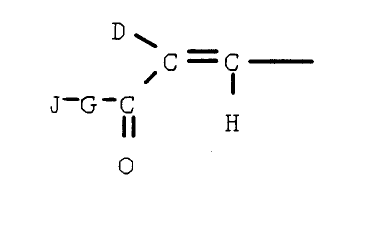 dans lequel D représente un atome d'hydrogène ou d'halogène, un radical alkyloxy renfermant de 1 à 8 atomes de carbone, G représente un atome d'oxygène ou de soufre et J représente ou bien un radical alkyle linéaire, ramifié ou cyclique, saturé ou insaturé, renfermant de 1 à 8 atomes de carbone, éventuellement substitué par un ou plusieurs groupements fonctionnels, identiques ou différents, ou bien un groupement aryle renfermant de 6 à 14 atomes de carbone, éventuellement substitué par un ou plusieurs groupements fonctionnels identiques ou différents, ou bien un radical hétérocyclique éventuellement substitué par un ou plusieurs groupements fonctionnels, identiques ou différents,

### ou bien A représente un radical :

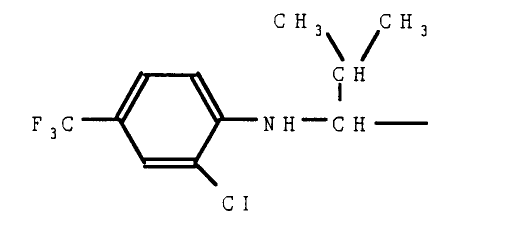

Lorsque X représente un radical alkyle, il s'agit de préférence du radical méthyle ou éthyle.

Lorsque X représente un radical alkényle, il s'agit de préférence du radical vinyle.

Lorsque X représente un radical alkynyle, il s'agit de préférence du radical éthynyle.

Lorsque Y représente un atome d'halogène, il s'agit de préférence d'un atome de fluor ou encore de chlore ou de brome.

Parmi les composés préférés de l'invention, on peut citer :
- les composés de formule (I) dans lesquels Y représente un radical fluor,
- les composés de formule (I) dans lesquels Y représente un radical CF₃,
- les composés de formule (I) dans lesquels X représente un atome d'hydrogène,
- les composés de formule (I) dans lesquels A représente un radical : 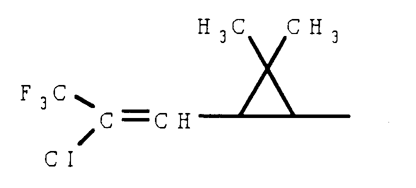
- les composés de formule (I) dans lesquels A représente un radical 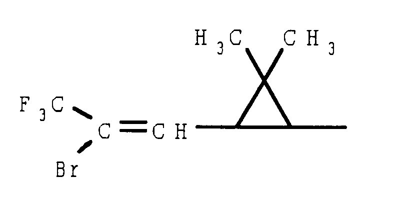
- les composés de formule (I) dans lesquels A représente un radical 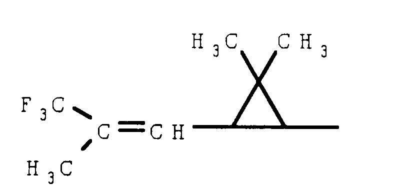
- les composés de formule (I) dans lesquels A représente le reste d'un acide cyclopropanique de structure 1R Cis.

Parmi les composés préférés de l'invention, on peut citer les composés dont la préparation est donnée ci-après dans la partie expérimentale.

L'invention a également pour objet un procédé de préparation caractérisé en ce que l'on soumet un acide de formule (II) :

ACO₂H (II)

dans laquelle A conserve sa signification précédente, ou un dérivé fonctionnel de cet acide, à l'action d'un alcool de formule (III) : 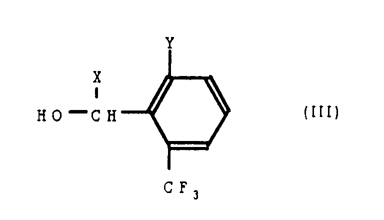 dans laquelle X et Y conservent leur signification précédente, ou d'un dérivé fonctionnel de cet alcool pour obtenir le composé de formule (I) correspondant.

Le dérivé fonctionnel d'acide utilisé est de préférence un chlorure d'acide.

Lorsque l'on fait réagir l'acide de formule (II) sur l'alcool de formule (III), on opère de préférence en présence de dicyclohexylcarbodiimide.

Les acides de formule (II) utilisés comme produits de départ sont en général des produits connus, utilisés dans la synthèse de composés pyréthrinoïdes.

Certains acides, dont la préparation est donnée ci-après dans la partie expérimentale sont nouveaux et l'invention a également pour objet ces acides à titre de produits industriels nouveaux.

Les alcools de formule (III) sont des produits connus d'une manière générale, l'alcool 2-fluor 6-(trifluorométhyl) benzylique est un produit commercial. L'alcool 2,6-bis(trifluorométhyl) benzylique est un produit nouveau dont la préparation est donnée ci-après dans la partie expérimentale. Cet alcool est un objet de la présente invention.

Les composés de formule (I) présentent d'intéressantes propriétés qui permettent leur utilisation dans la lutte contre les parasites. Il peut s'agir par exemple de la lutte contre les parasites des végétaux, les parasites des locaux et les parasites des animaux à sang chaud.

C'est ainsi que l'on peut utiliser les produits de l'invention pour lutter contre les insectes, les nématodes et les acariens parasites des végétaux et des animaux.

L'invention a notamment pour objet l'application des composés de formule (I) à la lutte contre les parasites des végétaux, les parasites des locaux et les parasites des animaux à sang chaud.

Les produits de formule (I) peuvent aussi être utilisés pour lutter contre les insectes et autres parasites du sol, par exemple les coléoptères, comme Diabrotica, les taupins et les vers blancs, les myriapodes comme les scutigérelles et les blaniules, et les diptères comme les cécydomies et les lépidoptères comme les noctuelles terricoles.

Ils sont utilisés à des doses comprises entre 10 g et 300 g de matière active à l'hectare.

Les produits de formule (I) peuvent également être utilisés pour lutter contre les insectes dans les locaux, pour lutter notamment contre les mouches, les moustiques et les blattes.

Les produits de formule (I) sont de plus photostables et sont peu toxiques pour les mammifères.

L'ensemble de ces propriétés fait que les produits de formule (I) correspondent parfaitement aux exigences de l'industrie agrochimique moderne : ils permettent de protéger les récoltes tout en préservant l'environnement.

Les produits de formule (I) peuvent aussi être utilisés pour lutter contre les acariens et les nématodes parasites des végétaux.

Les composés de formule (I) peuvent encore être utilisés pour lutter contre les acariens parasites des animaux, pour lutter par exemple contre les tiques et notamment les tiques de l'espèce de Boophilus, ceux de l'espèce Hyalomnia, ceux de l'espèce Amblyomnia et ceux de l'espèce Rhipicephalus ou pour lutter contre toutes sortes de gales et notamment la gale sarcoptique, la gale psoroptique et la gale chorioptique.

L'invention a donc également pour objet les compositions destinées à la lutte contre les parasites des animaux à sang chaud, les parasites des locaux et des végétaux, caractérisées en ce qu'elles renferment au moins l'un des produits de formule (I) définis ci-dessus et notamment les produits de formule (I) des exemples 1, 2, 3, 5 et 6.

L'invention a notamment pour objet les compositions insecticides renfermant comme principe actif au moins l'un des produits définis ci-dessus.

Ces compositions sont préparées selon les procédés usuels de l'industrie agrochimique ou de l'industrie vétérinaire ou de l'industrie des produits destinés à la nutrition animale.

Dans ces compositions destinées à l'usage agricole et à l'usage dans les locaux, la ou les matières actives peuvent être additionnées éventuellement d'un ou plusieurs autres agents pesticides. Ces compositions peuvent se présenter sous forme de poudres, granulés, suspensions, émulsions, solutions, solutions pour aérosols, bandes combustibles, appâts ou autres préparations employées classiquement pour l'utilisation de ce genre de composés.

Outre le principe actif, ces compositions contiennent, en général, un véhicule et/ou un agent tensio-actif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselguhr ou un solide combustible.

Les compositions insecticides selon l'invention contiennent de préférence de 0,005 % à 10 % en poids de matière active.

Selon un mode opératoire avantageux, pour un usage dans les locaux, les compositions selon l'invention sont utilisées sous forme de compositions fumigantes.

Les compositions selon l'invention peuvent alors être avantageusement constituées, pour la partie non active, d'un serpentin insecticide (ou coil) combustible, ou encore d'un substrat fibreux incombustible. Dans ce dernier cas, le fumigant obtenu après incorporation de la matière active est placé sur un appareil chauffant tel qu'un émanateur électrique.

Dans le cas où l'on utilise un serpentin insecticide, le support inerte peut être, par exemple, composé de marc de pyrèthre, poudre de Tabu (ou poudre de feuilles Machilus Thumbergii), poudre de tige de pyrèthre, poudre de feuille de cèdre, poudre de bois (telle que de la sciure de pin) amidon et poudre de coque de noix de coco.

La dose de matière active peut alors être, par exemple, de 0,03 à 1 % en poids.

Dans le cas où l'on utilise un support fibreux incombustible, la dose de matière active peut alors être, par exemple, de 0,03 à 95 % en poids.

Les compositions selon l'invention pour un usage dans les locaux peuvent aussi être obtenues en préparant une huile pulvérisable à base de principe actif, cette huile imbibant la mèche d'une lampe et étant alors soumise à la combustion.

La concentration du principe actif incorporé à l'huile est, de préférence, de 0,03 à 95 % en poids.

L'invention a également pour objet les compositions acaricides et nématicides renfermant comme principe actif au moins un des produits de formule (I) définie ci-dessus.

Les compositions insecticides selon l'invention, comme les compositions acaricides et nématicides, peuvent être additionnées éventuellement d'un ou plusieurs autres agents pesticides. Les compositions acaricides et nématicides peuvent se présenter notamment sous forme de poudre, granulés, suspensions, émulsions, solutions.

Pour l'usage acaricide, on utilise de préférence des poudres mouillables, pour pulvérisation foliaire, contenant de 1 à 80 % en poids de principe actif ou des liquides pour pulvérisation foliaire contenant de 1 à 500 g/l de principe actif. On peut également employer des poudres pour poudrages foliaires contenant de 0,05 à 3 % de matière active.

Pour l'usage nématicide, on utilise de préférence des liquides pour traitement des sols contenant de 300 à 500 g/l de principe actif.

Les composés acaricides et nématicides selon l'invention sont utilisés, de préférence, à des doses comprises entre 1 et 100 g de matière active à l'hectare.

Pour exalter l'activité biologique des produits de l'invention on peut les additionner à des synergistes classiques utilisés en pareil cas tel que le 1-(2,5,8-trioxadodécyl) 2-propyl 4,5-méthylènedioxy benzène (ou butoxyde de pipéronyle) ou la N-(2-éthyl heptyl) bicyclo[2,2-1]5-heptène-2,3-dicarboximide, ou le pipéronyl-bis-2-(2'-n-butoxy éthoxy) éthylacétal (ou tropital).

Les composés de formule (I) présentent une excellente tolérance générale, et l'invention a donc également pour objet les produits de formule (I), pour lutter notamment contre les affections créées par les tiques et les gales chez l'homme et l'animal.

Les produits de l'invention sont notamment utilisés pour lutter contre les poux à titre préventif ou curatif et pour lutter contre la gale.

Les produits de l'invention peuvent être administrés par voie externe, par vaporisation, par shampooing, par bain ou badigeonnage.

Les produits de l'invention à usage vétérinaire peuvent être également administrés par badigeonnage de l'épine dorsale selon la méthode dite méthode "pour-on".

On peut indiquer également que les produits de l'invention peuvent être utilisés comme biocides ou comme régulateurs de croissance.

L'invention a également pour objet les associations douées d'activité insecticide, acaricide ou nématicide, caractérisées en ce qu'elles contiennent comme matière active, d'une part un au moins des composés de formule générale (I), et d'autre part, un au moins des esters pyréthrinoïdes choisi dans le groupe constitué par les esters d'alléthrolone, d'alcool 3,4,5,6-tétrahydrophtalimido méthylique, d'alcool 5-benzyl 3-furyl méthylique, d'alcool 3-phénoxy benzylique et d'alcool alpha-cyano 3-phénoxy benzylique des acides chrysanthémiques, par les esters d'alcool 5-benzyl 3-furyl méthylique des acides 2,2-diméthyl 3-(2-oxo 3-tétrahydrothiophény-lidène méthyl) cyclopropanecarboxyliques, par les esters d'alcool 3-phénoxy benzylique et d'alcool alpha-cyano 3-phénoxy benzylique des acides 2,2-diméthyl 3-(2,2-dichlorovinyl) cyclopropanecarboxyliques, par les esters d'alcool alpha-cyano 3-phénoxy benzylique d'acides 2,2-diméthyl 3-(2,2-dibromovinyl) cyclopropanecarboxyliques, par les esters d'alcool 3-phénoxy benzylique des acides 2-parachlorophényl 2-isopropyl acétiques, par les esters d'alléthrolone, d'alcool 3,4,5,6-tétrahydrophtalimidométhylique, d'alcool 5-benzyl 3-furyl méthylique, d'alcool 3-phénoxy benzylique et d'alcool alpha-cyano 3-phénoxy benzylique des acides 2,2-diméthyl 3-(1,2,2,2-tétrahaloéthyl) cyclopropanecarboxyliques, dans lesquels "halo" représente un atome de fluor, de chlore ou de brome, étant entendu que les composés (I) peuvent exister sous toutes leurs formes stéréoisomères possibles de même que les copules acides et alcools des esters pyréthrinoïdes ci-dessus.

Les exemples suivants illustrent l'invention sans toutefois la limiter.

### EXEMPLE 1 : [1R,(1alpha, 3alpha)] 3-[(Z) 2-chloro 3,3,3-trifluoro 1-propényl] 2,2-diméthyl cyclopropanecarboxylate de [2,6-bis(trifluorométhyl) phényl] méthyle

On ajoute à 0°C, une solution renfermant 0,31 g de dicyclohexylcarbodiimide et 1,2 ml de chlorure de méthylène, dans un mélange renfermant 0,37 g d'acide [1R,(1alpha, 3alpha)] 3-[(Z) 2-chloro 3,3,3-trifluoro 1-propényl] 2,2-diméthyl cyclopropanecarboxylique, 5 ml de chlorure de méthylène, 0,37 g d'alcool 2,6-bis(trifluorométhyl) benzylique dont la préparation est donnée ci-après et 4 mg de diméthylaminopyridine. On maintient le mélange réactionnel sous agitation pendant 3 heures. On filtre, lave et amène à sec sous pression réduite. On chromatographie le produit obtenu sur silice (éluant hexane-acétate d'éthyle 95-5). On obtient ainsi 0,61 g de produit recherché.
- RMN : CDCl₃ - 300 MHz:
- H des méthyle géminés: 1,27 (s) et 1,29 (s) ppm
- H en 1 et 3 du cyclopropane: 1,95 (d) J = 8,5 Hz
et 2,16 (m) ppm
- H du CO₂CH₂-: 5,28 (d) et 5,46 (d) ppm
- H éthylénique: 6,90 (dd) ppm J = 9,5 et 1 Hz
- H aromatiques: 7,66 (t) ppm, 7,97 (d)

### EXEMPLE 2 : [1R,(1alpha, 3alpha)] 3-[(Z) 2-chloro 3,3,3-trifluoro 1-propényl] 2,2-diméthyl cyclopropanecarboxylate de [2-fluoro 6-(trifluorométhyl) phényl] méthyle

En opérant comme à l'exemple 1, à partir de 4,18 g d'acide [1R,(1alpha, 3alpha)] 3-[(Z) 2-chloro 3,3,3-trifluoro 1-propényl] 2,2-diméthyl cyclopropanecarboxylique et de 1 g d'alcool 2-fluoro 6-(trifluorométhyl) benzylique commercial, on obtient 5,59 g de produit recherché.
- RMN : CDCl₃ - 250 MHz:
- H des méthyle géminés: 1,27 (s) et 1,29 (s) ppm
- H en 1 et 3 du cyclopropane: 1,97 (d) J = 8,5 Hz
et 2,16 (m) ppm
- H du CO₂CH₂: 5,30 [AB]
- H éthylénique: 6,89 (d) ppm
- H aromatiques: 7,33 (m) et 7,53 (m) ppm

En opérant comme aux exemples 1 et 2 à partir des acides et des alcools correspondants, on a obtenu les produits suivants :

### EXEMPLE 3 : [1R(1alpha,3alpha)] 3-[(Z)-2-bromo 3,3,3-trifluoro 1-propényl] 2,2-diméthyl cyclopropane carboxylate de [2,6-bis-(trifluorométhyl) phényl] méthyle.

- RMN : CDCl₃ - 250 MHz:
- H des méthyle géminés: 1,27 et 1,30 ppm
- H en 1 et 3 du cyclopropane: 1,35 (d) J = 8,5 Hz
et 2,12 (m) ppm
- H du CO₂CH₂: 5,28 (d) J = 13
- H éthylénique ΔZ: 7,12 (d) ppm

### EXEMPLE 4 : [1R(1alpha,3alpha)] 3-[(Z)-2-bromo 3,3,3-trifluoro 1-propényl] 2,2-diméthyl cyclopropane carboxylate de [2-fluoro 6-(trifluorométhyl) phényl] méthyle.

- RMN : CDCl₃ - 250 MHz:
- H des méthyle géminés: 1,28 (s) et 1,29 (s) ppm
- H en 1 et 3 du cyclopropane: 1,98 (d) J = 8,5 Hz
et 2,11 (m) ppm
- H du CO₂CH₂: 5,30 ppm

### EXEMPLE 5 : [1R(1alpha,3alpha)] 3-[(Z)-2-méthyl 3,3,3-trifluoro 1-propényl] 2,2-diméthyl cyclopropane carboxylate de [2,6-bis (trifluorométhyl) phényl] méthyle.

- RMN : CDCl₃ - 250 MHz:
- H des méthyle géminés: 1,27 (s) et 1,23 (s) ppm
- CH₃-C=: 1,83 (s) ppm
- H en 1 et 3 du cyclopropane: 1,85 (m) ppm
- H du CO₂CH₂: 5,26 (d) et 5,44 (d) ppm

### EXEMPLE 6 : [1R(1alpha,3alpha)] 3-[(Z)-2-méthyl 3,3,3-trifluoro 1-propényl] 2,2-diméthyl cyclopropane carboxylate de [2-fluoro 6-(trifluorométhyl) phényl] méthyle.

- RMN : CDCl₃ - 250 MHz:
- H des méthyle géminés: 1,24 (s) et 1,27 (s) ppm
- CH₃-C=: 1,83 (s) ppm
- H en 1 et 3 du cyclopropane: 1,85 (m) ppm
- H du CO₂CH₂: 5,28 ppm

### PREPARATION 1 : Alcool 2,6-bis(trifluorométhyl) benzylique

### STADE A : 2,6-bis(trifluorométhyl) benzoate de méthyle

On agite pendant 30 minutes à 20°C, une solution renfermant 6 g d'acide 2,6-bis(trifluorométhyl) benzoïque, 60 cm³ de tétrahydrofuranne et 11,58 cm³ d'une solution de soude 2N. On refroidit à 0°C et ajoute 4,26 cm³ de sulfate de diméthyle. On agite 1 heure à 20°C et ajoute à nouveau 2,1 cm³ de sulfate de diméthyle et maintient le mélange réactionnel sous agitation pendant 24 heures à 20°C. On le verse sur une solution aqueuse de bicarbonate de sodium, on extrait à l'éther isopropylique puis à l'acétate d'éthyle. On sèche, filtre, rince et amène à sec. On obtient ainsi, après chromatographie sur silice (éluant hexane-acétate d'éthyle (9-1)) 5,59 g du produit recherché.

### STADE B : Alcool 2,6-bis(trifluorométhyl) benzylique

On ajoute à 0°C, 58 cm³ d'une solution 1,2 M d'hydrure de diisobutylaluminium (DIBAH) dans une solution renfermant 5,59 g du produit préparé au stade A et 60 cm³ de toluène. On laisse la température revenir à 20°C et maintient le mélange réactionnel sous agitation pendant 4 heures. On verse dans une solution molaire de tartrate double de potassium et de sodium. On extrait à l'éther isopropylique, sature la phase aqueuse avec du chlorure de sodium. On extrait à l'acétate d'éthyle, sèche, filtre, rince et amène à sec. Après chromatographie sur silice (éluant : hexane-acétate d'éthyle (9-1)) on obtient 4,72 g de produit recherché.

### PREPARATION 2 : Acide [1R(1alpha,3alpha)] 3-[(Z)-2-bromo 3,3,3-trifluoro 1-propényl] 2,2-diméthyl cyclopropane carboxylique.

On chauffe 1 heure à 45°C 1 g de [1R(1alpha,3alpha)] 2,2-diméthyl 3-(2,2-dibromoéthényl) cyclopropane carboxylate de méthyle, 0,57 cm³ de triméthyl trifluorométhylsilane et 0,22 g de fluorure de potassium dans 10 cm³ de diméthylformamide en présence de 0,79 g d'iodure de cuivre. On ajoute de nouveau une même quantité de réactifs, agite 16 heures à 50°C, verse sur une solution aqueuse glacée de phosphate acide de sodium, extrait à l'éther, élimine le solvant et obtient 1,3 g de produit attendu brut. Après chromatographie sur silice (éluant : hexane-chlorure de méthylène 8-2), on obtient 250 mg de produit pur.

### PREPARATION 3 : Acide [1R(1alpha,3alpha)] 2,2-diméthyl 3-[(Z)-2-méthyl 3,3,3-trifluoro 1-propényl] cyclopropane carboxylique.

### STADE A : 3-(2-oxopropyl) 2,2-diméthyl cyclopropane carboxylate de méthyle.

On chauffe à 30°C/34°C 10 g d'acide 3-(2-oxopropyl) 2,2-diméthyl cyclopropane carboxylique dans 100 cm³ d'acétone en présence de 7,35 g de carbonate acide de potassium et 5 cm³ de diméthylsulfate. Après 4 heures d'agitation, on ajoute 0,85 cm³ de diméthylsulfate et maintient 20 heures à 30°/34°C. On filtre, reprend à l'éther éthylique, évapore le solvant, chromatographie le résidu sur silice (éluant : hexane-acétate d'éthyle 8-2) et obtient 10,43 g de produit attendu.

### STADE B : 3-(2-méthyl 2-triméthylsilyloxy 3,3,3-trifluoropropyl) 2,2-diméthyl cyclopropane carboxylate de méthyle.

A 5 g de produit obtenu au stade A et 8 cm³ de trifluorométhyl triméthylsilane dans 65 cm³ de tétrahydrofuranne, on ajoute à 0°C, 0,5 cm³ de fluorure de terbutylammonium et agite 15 minutes. On verse dans une solution aqueuse glacée de phosphate acide de potassium, extrait à l'éther éthylique, sèche et évapore le solvant. On obtient 7,57 g de produit attendu.

### STADE C : 3-(2-méthyl 2-hydroxy 3,3,3-trifluoropropyl) 2,2-diméthyl cyclopropane carboxylate de méthyle.

On ajoute à température ambiante 444 mg de fluorure de potassium à 500 mg de produit obtenu au stade B dans 5 cm³ de méthanol. On agite 3 heures, verse dans 25 cm³ d'une solution aqueuse de phosphate acide de potassium, extrait à l'éther éthylique, sèche et évapore le solvant. On obtient 360 mg de produit attendu.

### STADE D : 2,2-diméthyl 3-[(Z) 2-méthyl 3,3,3-trifluoro 1-propényl] cyclopropane carboxylate de méthyle.

On ajoute à 20°C 22,3 cm³ d'oxychlorure de phosphore à 24,35 g de 2,2-diméthyl 3-(2-méthyl 2-hydroxy 3,3,3-trifluoropropyl) cyclopropane carboxylate de méthyle obtenu au stade C dans 146 cm³ de pyridine. On chauffe à 74°C pendant 6 heures et demie, laisse 16 heures à température ambiante, verse sur de l'eau glacée, extrait à l'éther isopropylique, lave la phase organique à l'acide chlorhydrique 2N puis à l'eau, sèche et évapore le solvant. Après chromatographie sur silice (éluant : hexane-acétate d'éthyle 75-25), on obtient 19,13 g de produit attendu.

### STADE E : Acide [1R(1alpha,3alpha)] 2,2-diméthyl 3-[(Z)-2-méthyl 3,3,3-trifluoro 1-propényl] cyclopropane carboxylique.

On chauffe à 60°C pendant 2 heures et demie 4,14 g de produit obtenu au stade A dans 52,6 cm³ de méthanol en présence de 19,3 cm³ de soude N puis 30 minutes en présence de 2 cm³ de soude N supplémentaires. On verse le milieu réactionnel dans l'eau glacée, extrait à l'éther isopropylique, sèche et évapore le solvant sous pression réduite. On obtient 2,94 g de produit attendu.

### EXEMPLE 7 : Préparation d'un concentré soluble

On effectue un mélange homogène de :

| | |
|---|---|
| Produit de l'exemple 1 | 0,25 g |
| Butoxyde pipéronyle | 1,00 g |
| Tween 80 | 0,25 g |
| Topanol A | 0,1 g |
| Eau | 98,4 g |

### EXEMPLE 8 : Préparation d'un concentré émulsifiable

On mélange intimement :

| | |
|---|---|
| Produit de l'exemple 2 | 0,015 g |
| Butoxyde de pipéronyle | 0,5 g |
| Topanol A | 0,1 g |
| Tween 80 | 3,5 g |
| Xylène | 95,885 g |

### EXEMPLE 9 : Préparation d'un concentré émulsifiable

On effectue un mélange homogène de :

| | |
|---|---|
| Produit de l'exemple 3 | 0,25 g |
| Butoxyde pipéronyle | 1,00 g |
| Tween 80 | 0,25 g |
| Topanol A | 0,1 g |
| Eau | 98,4 g |

### EXEMPLE 10 : Préparation d'un concentré émulsifiable

On mélange intimement :

| | |
|---|---|
| Produit de l'exemple 5 | 0,015 g |
| Butoxyde de pipéronyle | 0,5 g |
| Topanol A | 0,1 g |
| Tween 80 | 3,5 g |
| Xylène | 95,885 g |

### EXEMPLE 11 : Préparation d'un concentré émulsifiable

On effectue un mélange homogène de :

| | |
|---|---|
| Produit de l'exemple 6 | 1,5 g |
| Tween 80 | 20,00 g |
| Topanol A | 0,1 g |
| Eau | 78,4 g |

### EXEMPLE 12 : Préparation de granulés

On a préparé des granulés contenant de 0,1 % à 5 % de substances actives.

### ETUDE BIOLOGIQUE

### A - Activité sur Diabrotica

Les insectes tests sont des larves de dernier stade de Diabrotica. On traite une rondelle de papier filtre de 9 cm de diamètre, déposée au fond d'une boite de Pétri, à l'aide de 2 cm³ de solution acétonique du produit à tester. Après séchage on dépose 15 larves par dose et on effectue le contrôle de mortalité 24 heures après le traitement.

Dès la dose de 1 ppm les produits de l'invention présentent une bonne activité.

### B - Etude de l'effet d'abattage sur mouche domestique

Les insectes tests sont des mouches domestiques femelles âgées de 4 jours. On opère par pulvérisation en chambre de Kearns et March en utilisant comme solvant un mélange d'acétone (5 %) et d'Isopar L (solvant pétrolier) (quantité de solvant utilisée 2 ml en une seconde). On utilise 50 insectes par traitement. On effectue les contrôles toutes les minutes jusqu'à 10 minutes, puis à 15 minutes et l'on détermine le KT 50 par les méthodes habituelles.

A la dose de 1 g/l le produit de l'exemple 2 présente une bonne activité.

### C - Etude de l'effet létal sur mouche domestique

Les insectes tests sont des mouches domestiques femelles âgées de 4 à 5 jours. On opère par application topique de 1 microlitre de solution acétonique du produit à tester sur le thorax dorsal des insectes à l'aide du micro manipulateur d'Arnold. On utilise 50 individus par traitement. On effectue le contrôle de mortalité vingt-quatre heures après traitement.

A la dose de 10 mg/l le produit de l'exemple 2 présente une bonne activité.

## Revendications

1. Sous toutes les formes stéréoisomères possibles ainsi que leurs mélanges, les composés de formule (I) : 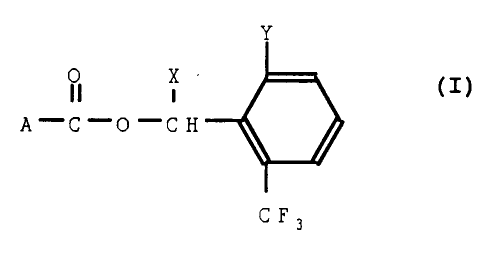 dans laquelle :
- X représente un atome d'hydrogène, un radical alkyle, alkényle ou alkynyle renfermant jusqu'à 4 atomes de carbone ou un radical cyano ou un radical aralkynyle renfermant jusqu'à 10 atomes de carbone,
Y représente un atome d'halogène ou un radical CH₂F, CHF₂ ou CF₃,
ou bien A représente un radical : 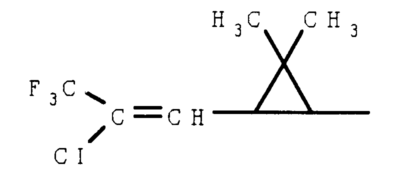 ou bien A représente un radical : 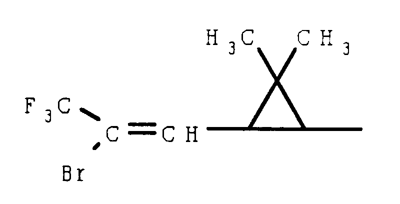 ou bien A représente un radical :

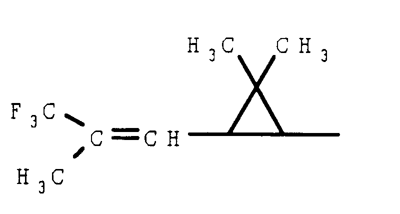

2. Les composés de formule (I) tels que définis à la revendication 1, dans lesquels Y représente un radical fluor.

3. Les composés de formule (I) tels que définis à la revendication 1 dans lesquels Y représente un radical CF₃.

4. Les composés de formule (I) tels que définis à l'une quelconque des revendications 1 à 3, dans lesquels X représente un atome d'hydrogène.

5. Les composés de formule (I) tels que définis à l'une quelconque des revendications 1 à 4, dans lesquels A représente un radical :

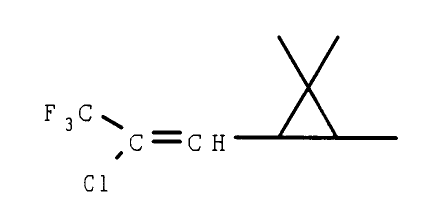

6. Les composés de formule (I) tels que définis à l'une quelconque des revendications 1 à 5, dans lesquels A représente le reste d'un acide cyclopropanique de structure 1R Cis.

7. Les composés de formule (I) définis à la revendication 1, dont les noms suivent :
- le [1R,(1alpha, 3alpha)] 3-[(Z) 2-chloro 3,3,3-trifluoro 1-propényl] 2,2-diméthyl cyclopropanecarboxylate de [2,6-bis(trifluorométhyl) phényl] méthyle,
- le [1R,(1alpha, 3alpha)] 3-[(Z) 2-chloro 3,3,3-trifluoro 1-propényl] 2,2-diméthyl cyclopropanecarboxylate de [2-fluoro 6-(trifluorométhyl) phényl] méthyle,
- le [1R(1alpha,3alpha)] 3-[(Z)-2-bromo 3,3,3-trifluoro 1-propényl] 2,2-diméthyl cyclopropane carboxylate de [2,6-bis-(trifluorométhyl) phényl] méthyle,
- le [1R(1alpha,3alpha)] 3-[(Z)-2-bromo 3,3,3-trifluoro 1-propényl] 2,2-diméthyl cyclopropane carboxylate de [2-fluoro 6-(trifluorométhyl) phényl] méthyle,
- le [1R(1alpha,3alpha)) 3-[(E)-2-méthyl 3,3,3-tri-fluoro 1-propényl] 2,2-diméthyl cyclopropane carboxylate de [2,6-bis-(trifluorométhyl) phényl] méthyle,
- le [1R(1alpha,3alpha)] 3-[(E)-2-méthyl 3,3,3-tri-fluoro 1-propényl] 2,2-diméthyl cyclopropane carboxylate de [2-fluoro 6-(trifluorométhyl) phényl] méthyle.

8. Procédé de préparation des composés de formule (I) tels que définis à l'une quelconque des revendications 1 à 7, caractérisé en ce que l'on soumet un acide de formule (II) :
ACO₂H (II)
dans laquelle A conserve sa signification précédente, ou un dérivé fonctionnel de cet acide, à l'action d'un alcool de formule (III) : 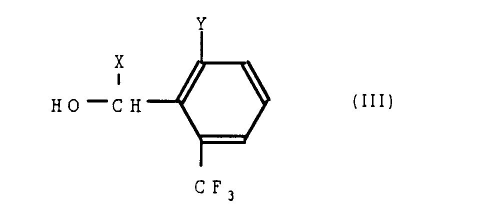 dans laquelle X et Y conservent leur signification précédente, ou d'un dérivé fonctionnel de cet alcool pour obtenir le composé de formule (I) correspondant.

9. A titre de produit chimique nouveau, l'alcool de formule (III) dont le nom suit :
- l'alcool 2,6-bis(trifluorométhyl) benzylique.

10. A titre de produits chimiques nouveaux, les acides de formule (II) dont les noms suivent :
- l'acide [1R(1alpha,3alpha)] 3-[(Z)-2-bromo 3,3,3-trifluoro 1-propényl] 2,2-diméthyl cyclopropane carboxylique,
- l'acide [1R(1alpha,3alpha)] 2,2-diméthyl 3-[(Z)-2-méthyl 3,3,3-trifluoro 1-propényl] cyclopropane carboxylique.

11. Application des composés de formule (I) tels que définis à l'une quelconque des revendications 1 à 7, à la lutte contre les parasites des végétaux et les parasites des locaux.

12. Les compositions destinées à la lutte contre les parasites des végétaux, les parasites des locaux et les parasites des animaux à sang chaud, caractérisées en ce qu'elles renferment comme principe actif au moins un composé défini à l'une quelconque des revendications 1 à 7.

13. Les compositions insecticides, caractérisées en ce qu'elles renferment comme principe actif au moins un composé défini à l'une quelconque des revendications 1 à 7

14. Les compositions insecticides définies à la revendication 12, caractérisées en ce qu'elles sont destinées à la lutte contre DIABROTICA et les autres parasites du sol.

15. Les compositions acaricides, caractérisées en ce qu'elles renferment comme principe actif au moins un composé défini à l'une quelconque des revendications 1 à 7.

16. Les compositions définies à l'une quelconque des revendications 14 à 17 caractérisées en ce qu'elles renferment comme principe actif au moins un composé défini à la revendication 7.

17. Associations douées d'activité insecticide, acaricide ou nématiside, caractérisées en ce qu'elles contiennent comme matière active, d'une part, un au moins des composés de formule générale (I) et d'autre part, un au moins des esters pyréthrinoïdes choisis dans le groupe constitué par les esters d'alléthrolone, d'alcool 3,4,5,6-tétrahydrophtalimido méthylique, d'alcool 5-benzyl 3-furyl méthylique, d'alcool 3-phénoxy benzylique et d'alcool alpha-cyano 3-phénoxy benzylique des acides chrysanthémiques, par les esters d'alcool 5-benzyl 3-furyl méthylique des acides 2,2-diméthyl 3-(2-oxo 3-tétrahydrothiophénylidène méthyl) cyclopropanecarboxyliques, par les esters d'alcool 3-phénoxy benzylique et d'alcool alpha-cyano 3-phénoxy benzylique des acides 2,2-diméthyl 3-(2,2-dichlorovinyl) cyclopropanecarboxyliques, par les esters d'alcool alpha-cyano 3-phénoxy benzylique d'acides 2,2-diméthyl 3-(2,2-dibromovinyl) cyclopropanecarboxyliques, par les esters d'alcool 3-phénoxy benzylique des acides 2-parachlorophényl 2-isopropyl acétiques, par les esters d'alléthrolone, d'alcool 3,4,5,6-tétrahydrophtalimidométhylique, d'alcool 5-benzyl 3-furyl méthylique, d'alcool 3-phénoxy benzylique et d'alcool alpha-cyano 3-phénoxy benzylique des acides 2,2-diméthyl 3-(1,2,2,2-tétrahaloéthyl) cyclopropanecarboxyliques, dans lesquels "halo" représente un atome de fluor, de chlore ou de brome, étant entendu que les composés (I) peuvent exister sous toutes leurs formes stéréoisomères possibles de même que les copules acides et alcools des esters pyréthrinoïdes ci-dessus.

## Claims

1. In all the possible stereoisomer forms, as well as their mixtures, the compounds of formula (I): 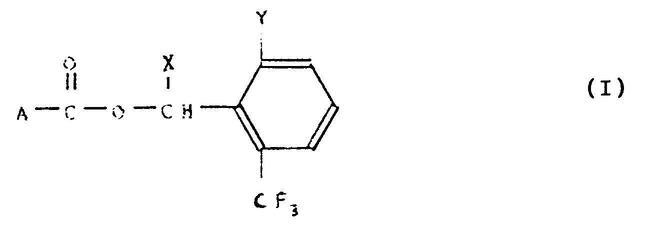 in which:
- X represents a hydrogen atom, an alkyl, alkenyl or alkynyl radical containing up to 4 carbon atoms or a cyano radical or an aralkynyl radical containing up to 10 carbon atoms,
Y represents a halogen atom or a CH₂F, CHF₂ or CF₃ radical,
either A represents a radical: 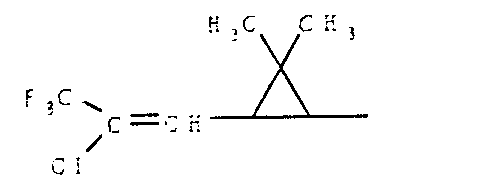 or A represents a radical: 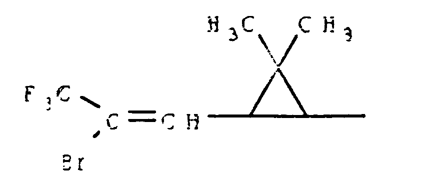 or A represents a radical:

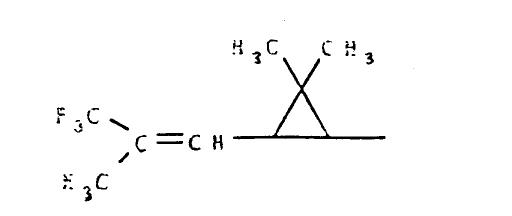

2. The compounds of formula (I) as defined in claim 1, in which Y represents a fluorine radical.

3. The compounds of formula (I) as defined in claim 1, in which Y represents a CF₃ radical.

4. The compounds of formula (I) as defined in any one of claims 1 to 3, in which X represents a hydrogen atom.

5. The compounds of formula (I) as defined in any one of claims 1 to 4, in which A represents a radical:

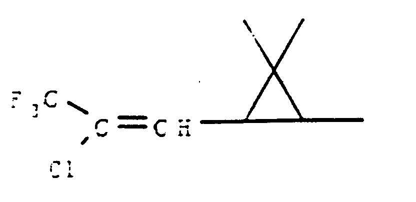

6. The compounds of formula (I) as defined in any one of claims 1 to 5, in which A represents the remainder of a cyclopropanic acid of 1R cis structure.

7. The compounds of formula (I) defined in claim 1, of which the names follow:
- [2,6-bis(trifluoromethyl) phenyl] methyl [1R,(1alpha, 3alpha)] 3-[(Z) 2-chloro 3,3,3-trifluoro 1-propenyl] 2,2-dimethyl cyclopropane carboxylate,
- [2-fluoro 6-(trifluoromethyl) phenyl] methyl [1R,(1alpha, 3alpha)] 3-[(Z) 2-chloro 3,3,3-trifluoro 1-propenyl] 2,2-dimethyl cyclopropane carboxylate,
- [2,6-bis(trifluoromethyl) phenyl] methyl [1R(1alpha, 3alpha)] 3-[(Z)-2-bromo 3,3,3-trifluoro 1-propenyl] 2,2-dimethyl cyclopropane carboxylate,
- [2-fluoro 6-(trifluoromethyl) phenyl] methyl [1R(1alpha, 3alpha)] 3-[(Z)-2-bromo 3,3,3-trifluoro 1-propenyl] 2,2-dimethyl cyclopropane carboxylate,
- [2,6-bis(trifluoromethyl) phenyl] methyl [1R(1alpha, 3alpha)] 3-[(E)-2-methyl 3,3,3-trifluoro 1-propenyl] 2,2-dimethyl cyclopropane carboxylate,
- [2-fluoro 6-(trifluoromethyl) phenyl] methyl [1R(1alpha, 3alpha)] 3-[(E)-2-methyl 3,3,3-trifluoro 1-propenyl] 2,2-dimethyl cyclopropane carboxylate.

8. Preparation process for the compounds of formula (I) as defined in any one of claims 1 to 7, characterized in that an acid of formula (II):
ACO₂H (II)
in which A keeps its previous meaning, or a functional derivative of this acid, is subjected to the action of an alcohol of formula (III): 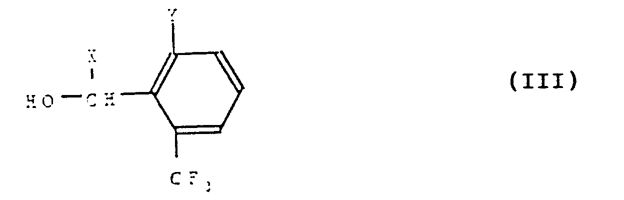 in which X and Y keep their previous meaning, or a functional derivative of this alcohol, in order to obtain the corresponding compound of formula (I).

9. As a new chemical product, the alcohol of formula (II) the name of which follows:
- 2,6-bis(trifluoromethyl) benzyl alcohol.

10. As new chemical products, the acids of formula (II) of which the names follow:
- [1R(1alpha,3alpha)] 3-[(Z)-2-bromo 3,3,3-trifluoro 1-propenyl] 2,2-dimethyl cyclopropane carboxylic acid,
- [1R(1alpha,3alpha)] 2,2-dimethyl 3-[(Z)-2-methyl 3,3,3-trifluoro 1-propenyl] cyclopropane carboxylic acid.

11. Use of the compounds of formula (I) as defined in any one of claims 1 to 7, for combating parasites of vegetation and parasites of premises.

12. The compositions intended for combating parasites of vegetation, parasites of premises and parasites of warm-blooded animals, characterized in that they contain as active ingredient at least one compound defined in any one of claims 1 to 7.

13. The insecticide compositions, characterized in that they contain as active ingredient at least one compound defined in any one of claims 1 to 7.

14. The insecticide compositions defined in claim 12, characterized in that they are intended for combating DIABROTICA and other soil parasites.

15. The acaricide compositions, characterized in that they contain as active ingredient at least one compound defined in any one of claims 1 to 7.

16. The compositions defined in any one of claims 14 to 17 characterized in that they contain as active ingredient at least one compound defined in claim 7.

17. Combinations endowed with insecticide, acaricide or nematicide activity, characterized in that they contain as active ingredient, on the one hand at least one of the compounds of general formula (I), and on the other hand at least one of the pyrethrinoid esters chosen from the group constituted by the esters of allethrolone, of 3,4,5,6-tetrahydrophthalimidomethyl alcohol, of 5-benzyl-3-furyl methyl alcohol, of 3-phenoxybenzyl alcohol and of alpha-cyano-3-phenoxybenzyl alcohol with chrysanthemic acids, by the esters of 5-benzyl-3-furyl methyl alcohol with 2,2-dimethyl-3-(2-oxo-3-tetrahydrothiophenylidenemethyl)-cyclopropanecarboxylic acids, by the esters of 3-phenoxybenzyl alcohol and of alpha-cyano-3-phenoxybenzyl alcohol with 2,2-dimethyl-3-(2,2-dichlorovinyl)-cyclopropane-carboxylic acids, by the esters of alpha-cyano-3-phenoxybenzyl alcohol with 2,2-dimethyl-3-(2,2-dibromovinyl)-cyclopropanecarboxylic acids, by the esters of 3-phenoxybenzyl alcohol with 2-parachlorophenyl-2-isopropyl acetic acids, by the esters of allethrolone, of 3,4,5,6-tetrahydrophthalimidomethyl alcohol, of 5-benzyl-3-furyl methyl alcohol, of 3-phenoxybenzyl alcohol and of alphacyano-3-phenoxybenzyl alcohol with 2,2-dimethyl-3-(1,2,2,2-tetrahaloethyl)-cyclopropanecarboxylic acids, in which "halo" represents a fluorine, chlorine or bromine atom, it being understood that the compounds (I) can exist in all their possible stereoisomer forms, as well as the acid and alcohol copulas of the above pyrethrinoid esters.

## Patentansprüche

1. In allen möglichen stereoisomeren Formen sowie ihren Mischungen, die Verbindungen der Formel (I) 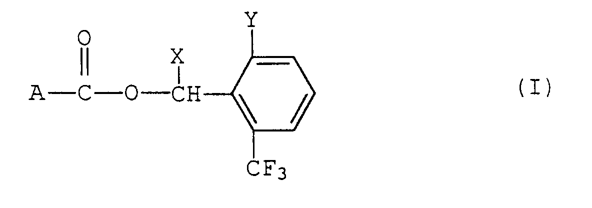 in der
- X ein Wasserstoffatom, einen Alkyl-, Alkenyl- oder Alkinylrest mit bis zu 4 Kohlenstoffatomen oder einen Cyanorest oder einen Aralkinylrest mit bis zu 10 Kohlenstoffatomen darstellt,
- Y ein Halogenatom oder einen Rest CH₂F, CHF₂ oder CF₃ bedeutet,
- A entweder einen Rest 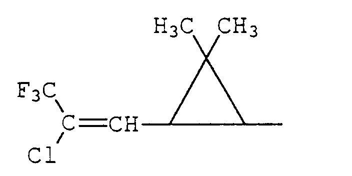
oder einen Rest 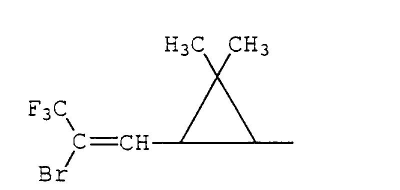 oder einen Rest 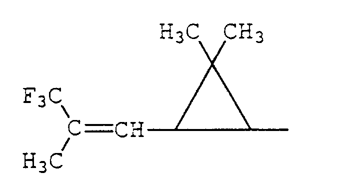 darstellt.

2. Verbindungen der Formel (I), wie in Anspruch 1 definiert, worin Y einen Fluorrest darstellt.

3. Verbindungen der Formel (I), wie in Anspruch 1 definiert, worin Y einen Rest CF₃ darstellt.

4. Verbindungen der Formel (I), wie in irgendeinem der Ansprüche 1 bis 3 definiert, worin X ein Wasserstoffatom darstellt.

5. Verbindungen der Formel (I), wie in irgendeinem der Ansprüche 1 bis 4 definiert, worin A einen Rest 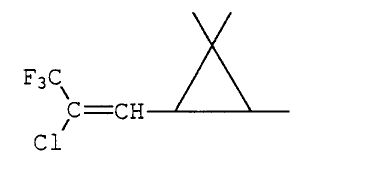 darstellt.

6. Verbindungen der Formel (I), wie in irgendeinem der Ansprüche 1 bis 5 definiert, worin A den Rest einer Cyclopropansäure der Struktur 1R cis darstellt.

7. Verbindungen der Formel (I), wie in Anspruch 1 definiert, mit den folgenden Namen:
- [1R,(1α,3α)]-3-[(Z)2-Chlor-3,3,3-trifluor-1-propenyl]-2,2-dimethyl-cyclopropancarbonsäure-[2,6-bis-(trifluormethyl)-phenyl]-methylester,
- [1R,(1α,3α)]-3-[(Z)2-Chlor-3,3,3-trifluor-1-propenyl]-2,2-dimethyl-cyclopropancarbonsäure-[2-fluor-6-(trifluormethyl)-phenyl]-methylester,
- [1R,(1α,3α)]-3-[(Z)2-Brom-3,3,3-trifluor-1-propenyl]-2,2-dimethyl-cyclopropancarbonsäure-[2,6-bis-(trifluormethyl)-phenyl]-methylester,
- [1R,(1α,3α)]-3-[(Z)2-Brom-3,3,3-trifluor-1-propenyl]-2,2-dimethyl-cyclopropancarbonsäure-[2-fluor-6-(trifluormethyl)-phenyl]-methylester,
- [1R,(1α,3α)]-3-[(E)2-Methyl-3,3,3-trifluor-1-propenyl]-2,2-dimethyl-cyclopropancarbonsäure-[2,6-bis-(trifluormethyl)-phenyl]-methylester,
- [1R,(1α,3α)]-3-[(E)2-Methyl-3,3,3-trifluor-1-propenyl]-2,2-dimethyl-cyclopropancarbonsäure-[2-fluor-6-(trifluormethyl)-phenyl]-methylester.

8. Verfahren zur Herstellung der Verbindungen der Formel (I), wie in irgendeinem der Ansprüche 1 bis 7 definiert, dadurch gekennzeichnet, daß man eine Säure der Formel (II)
ACO₂H (II)
in der A seine obige Bedeutung behält, oder ein funktionelles Derivat dieser Säure der Einwirkung eines Alkohols der Formel (III) 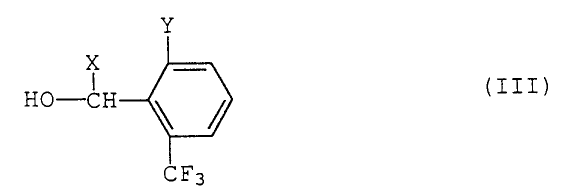 in der X und Y ihre obige Bedeutung behalten, oder eines funktionellen Derivates dieses Alkohols unterzieht, um die entsprechende Verbindung der Formel (I) zu erhalten.

9. Als neues chemisches Produkt der Alkohol der Formel (III) mit dem folgenden Namen:
- 2,6-Bis-(Trifluormethyl)-benzylalkohol.

10. Als neue chemische Produkte die Säuren der Formel (II) mit den folgenden Namen:
- [1R,(1α,3α)]-3-[(Z)2-Brom-3,3,3-trifluor-1-propenyl]-2,2-dimethyl-cyclopropancarbonsäure,
- [1R,(1α,3α)]-3-[(Z)2-Methyl-3,3,3-trifluor-1-propenyl]-2,2-dimethyl-cyclopropancarbonsäure.

11. Verwendung der Verbindungen der Formel (I), wie in irgendeinem der Ansprüche 1 bis 7 definiert, zur Bekämpfung von Parasiten an Pflanzen und Parasiten in Räumen.

12. Zusammensetzungen für die Bekämpfung von Parasiten an Pflanzen, Parasiten in Räumen und Parasiten an Warmblüter-Tieren, dadurch gekennzeichnet, daß sie als Wirkstoff mindestens eine Verbindung, wie in irgendeinem der Ansprüche 1 bis 7 definiert, umfassen.

13. Insektizide Zusammensetzungen, dadurch gekennzeichnet, daß sie als Wirkstoff mindestens eine Verbindung, wie in irgendeinem der Ansprüche 1 bis 7 definiert, umfassen.

14. Insektizide Zusammensetzungen wie in Anspruch 12 definiert, dadurch gekennzeichnet, daß sie für die Bekämpfung von DIABROTICA und anderen Bodenparasiten vorgesehen sind.

15. Akarizide Zusammensetzungen, dadurch gekennzeichnet, daß sie als Wirkstoff mindestens eine Verbindung, wie in irgendeinem der Ansprüche 1 bis 7 definiert, umfassen.

16. Zusammensetzungen wie in irgendeinem der Ansprüche 14 bis 17 definiert, dadurch gekennzeichnet, daß sie als Wirkstoff mindestens eine Verbindung, wie in Anspruch 7 definiert, umfassen.

17. Assoziationen mit insektizider, akarizider oder nematizider Wirkung, dadurch gekennzeichnet, daß sie als Wirkstoff einerseits mindestens eine der Verbindungen der allgemeinen Formel (I), und andererseits mindestens einen Pyrethrinoid-ester enthalten, gewählt aus der durch die Ester von Allethrolon, von 3,4,5,6-Tetrahydrophthalimido-methylalkohol, von 5-Benzyl-3-furyl-methylalkohol, von 3-Phenoxy-benzylalkohol und von α-Cyano-3-phenoxy-benzylalkohol mit Chrysanthemsäuren, durch die Ester von 5-Benzyl-3-furyl-methylalkohol mit 2,2-Dimethyl-3-(2-oxo-3-tetrahydrothiophenyliden-methyl)-cyclopropancarbonsäuren, durch die Ester von 3-Phenoxy-benzylalkohol und von α-Cyano-3-phenoxy-benzylalkohol mit 2,2-Dimethyl-3-(2,2-dichlorvinyl)-cyclopropancarbonsäuren, durch die Ester von α-Cyano-3-phenoxy-benzylalkohol mit 2,2-Dimethyl-3-(2,2-dibromvinyl)-cyclopropancarbonsäuren, durch die Ester von 3-Phenoxy-benzylalkohol mit 2-para-Chlorphenyl-2-isopropyl-essigsäuren, durch die Ester von Allethrolon, von 3,4,5,6-Tetrahydrophthalimido-methylalkohol, von 5-Benzyl-3-furyl-methyl-alkohol, von 3-Phenoxy-benzylalkohol und von α-Cyano-3-phenoxybenzylalkohol mit 2,2-Dimethyl-3-(1,2,2,2-tetrahaloethyl)-cyclopropancarbonsäuren, worin "Halo" ein Atom von Fluor, Chlor oder Brom darstellt, gebildeten Gruppe, mit der Maßgabe, daß die Verbindungen (I) in allen möglichen stereoisomeren Formen existieren können, ebenso wie die Paare von Säuren und Alkoholen der oben genannten Pyrethrinoid-ester.
